Europäisches Patentamt
European Patent Office
Office européen des brevets

(11) Veröffentlichungsnummer: 0 290 959
A2

(12) # EUROPÄISCHE PATENTANMELDUNG

(21) Anmeldenummer: 88107223.5

(22) Anmeldetag: 05.05.88

(51) Int. Cl.4: A61K 31/55 , A61K 31/635 , //(A61K31/55,31:505,31:49, 31:47),(A61K31/635,31:55)

Patentansprüche für folgende Vertragsstaaten: ES + GR.

(30) Priorität: 08.05.87 DE 3715378

(43) Veröffentlichungstag der Anmeldung: 17.11.88 Patentblatt 88/46

(84) Benannte Vertragsstaaten: AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Chatterjee, Dipak Kumar, Dr.
G-21, Hoechst Exec. Quarters P.O. Mulund Colony
Darga Road Bombay 400 082(IN)
Erfinder: Venugopalan, Bindumadhavan, Dr.
Bldg 43/A, Flat No. 31 Brindavan Housing Complex
P.O. Thane Maharashtra(IN)
Erfinder: Lal, Bansi, Dr.
30, Advani Apartments Mulund (West)
Bombay 400 080(IN)
Erfinder: de Souza, Noel John Dr.
Melrose, 62 Pali Hill Bandra
Bombay 400 050(IN)
Erfinder: Rupp, Richard Helmut, Dr.
Roederweg 16a
D-6240 Königstein/Taunus(DE)

(54) **Arzneimittelkombination zur Prophylaxe und Therapie von Malaria.**

(57) Die vorliegende Erfindung betrifft Kombinationen der Malariatherapeutika Artemisinin, Dihydroartemisinin, Arteether, Artemether, Artesunat oder anderer Artemisininderivate mit einem oder mehreren der Malariamittel Chloroquin, 10-O-Methylfloxacrin, Chinin, Mefloquin, Amodiaquin, Pyrimethamin, Sulfadoxin und Primaquin. Dabei werden der Behandlung von Säugern einschließlich des Menschen mit subkurativen Dosen der Einzelsubstanzen synergistische Wirkungen erzielt.

EP 0 290 959 A2

Xerox Copy Centre

## Arzneimittelkombination zur Prophylaxe und Therapie von Malaria

Die vorliegende Erfindung befaßt sich mit einer Kombination der Malariatherapeutika Artemisinin und seiner Derivate, z. B. Dihydroartemisinin, Arteether, Artemether oder Artesunat mit einem oder mehreren der Malariamittel Chloroquin, 10-O-Methylfloxacrin, Chinin, Mefloquin, Amodiaquin, Pyrimethamin, Sulfadoxin, Primaquin und deren pharmazeutisch verwendbaren Salzen zur Wirkungspotenzierung sowie mit Dosierungsmethoden dieser Wirkstoffkombinationen.

Die hier und an anderen Stellen des Textes benutzten generischen Bezeichnungen sind aus "Tropical Diseases Research, Seventh Programme Report", Kapitel 2; Malaria, UNDP·WORLD BANK/WHO, herausgegeben von der WHO 1985 entnommen; 10-O-Methylfloxacrin ist ein Derivat des Malariamittels Floxacrin und in der Deutschen Patentanmeldung P 36 24 778.2 beschrieben worden.

Die gegen Medikamente resistente Malaria stellt ein ernstes Problem für Klinik und öffentliche Gesundheitspflege dar. Der Malariaparasit Plasmodium falciparum hat eine vielseitige Fähigkeit entwickelt, entweder durch genetische Mechanismen oder nicht-genetische (adaptive) Methoden der Wirkung eines Medikamentes auszuweichen. Es ist nachgewiesen worden, daß es sich bei der Chloroquin-Resistenz von Malariaparasiten um eine stabile, genetisch determinierte Eigenschaft handelt (Warhurst, D.C. Pharmaceut. J. Nov. 23 (1985) 689-692). Die Ausbreitung von Plasmodium falciparum, die gegen Chloroquin und andere Malariamittel resistent sind, stellt das Programm der öffentlichen Gesundheitspflege in tropischen und subtropischen Ländern vor eine schwere Aufgabe (Suphat et al., Trans. R. Soc. Trop. Med. and Hyg. (1983) 73 (3), 338-340.

Die Verwendung von Kombination verschiedener Malariamittel ist in der Chemotherapie der Malaria bekannt. So ist beispielsweise in der Klinik eine Kombination von Amodiaquin und Tetracyclin und eine Kombination von Pyrimethamin und Sulfadoxin, die unter den Namen Fansidar® bekannt ist, eingesetzt worden (Suphat et al. a.a.O). In jüngerer Zeit werden mit einer weiteren Malariamittel-Kombination Fansimef (Mefloquin, Pyrimethamin und Sulfadoxin) klinische Studien angestellt (WHO a.a.O)

In einer Tierstudie hat Peters (Peters et al., Ann. Trop. Med. and Parasit. 71 (1977), 407-418) berichtet, daß die Resistenzentwicklung verlangsamt werden kann, wenn ein Malariamittel in Kombination mit bestimmten anderen Malariamitteln verabreicht wird. Peters (W. Peters, Bull. W.H.O. 51 (1974), 379-383 und W. Peters, Handbook of Experimented Pharmacology 68/11 (1984) Springer Verlag Berlin, Heidelberg, New York, Herausgeber: W. Peters und W.H.G. Richards) hat auch die Anwendung zweckmäßiger Medikamentenkombinationen für die Malariabehandlung beim Menschen hervorgehoben, die nicht nur die Resistenzentstehung hinauszögern können, sondern den Erfolg der Behandlung verbessern. So ist beispielsweise nachgewiesen worden, daß eine Dreierkombination aus Mefloquin, Sulfadoxin und Pyrimethamin die Resistenzentstehung bei Plasmodium berghei (Merkli et al., Ann. Trop. Med. and Parasit. (1980), 4(1), 1-9) hinauszögerte.

Artemisinin und Derivate sind als Malariamittel ebenfalls bereits bekannt. Artemisinin ist aus Artemisia annual L. isoliert, später synthetisiert und bei der Behandlung der P. falciparum-Malaria eingesetzt worden (H.P. Koch, Pharm. Int. (1981), 184-185; L.J. Bruce-Chwatt, British Med. J. 284 (1982), 767-768). Es hat sich auch gegen Chloroquinresistente Stäme von P. falciparum beim Menschen als wirksam erwiesen. Dihydroartemisinin, Arteether, Artemether und Artesunat z.B. sind halbsynthetische Derivate von Artemisinin, deren Wirkung gegen Malaria in verschiedenen Berichten beschrieben wird. (W.H.O. Report of the Scientific Working Group on the Chemotherapy of Malaria, PDR/Chemal 3rd Review 85.3, Geneva, 3-5 June 1985 und darin enthaltene Literaturstellen).

Artemisinin und seine Derivate werden durch Formel I dargestellt,

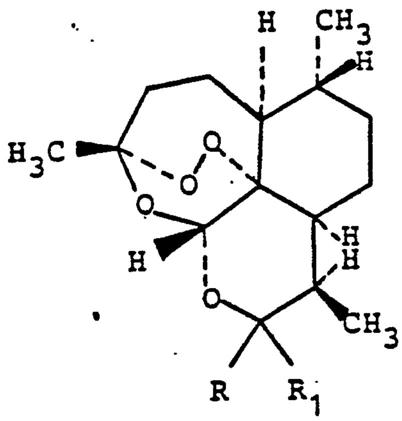

I

wobei R und $R_1$ zusammen Sauerstoff (Artemisinin), oder R jeweils Wasserstoff und $R_1$ OH (Dihydroartemisinin), -O-$C_1$-$C_6$-Alkyl, -O-$C_1$-$C_6$-Alkenyl, -O-$C_1$-$C_6$-Alkanoyl, -O-$C_1$-$C_6$-Alkanoylcarbonsäure, -O-Cyclohexylcarbonyl, -O-Benzoyl oder -O-Naphtoyl bedeutet, sowie die entsprechenden pharmakologisch verträglichen Salze,

Die Verbindung der Formel I mit R = H und $R_1$ = -O-$CH_3$ wird als Artemether, bei R = H und $R_1$ = -O-$C_2H_5$ als Arteether, bei R = H und $R_1$ = -O-$COCH_2CH_2CO_2Na$ als Artesunat bezeichnet.

Überraschenderweise wurde nun gefunden, daß Kombinationen von Artemisinin und/oder seiner oben genannten Derivate mit den bekannten Malariatherapeutika Chloroquin, 10-O-Methylfloxacrin, Chinin, Mefloquin, Amodiaquin, Pyrimethamin, Sulfadoxin, Primaquin und deren pharmazeutisch verwendbaren Salzen synergistische Wirkung zeigen.

Die klinische Bedeutung der vorliegenden verbesserten Zusammensetzung für die Malariatherapie speigeln entsprechende Tierexperimente wieder. In den nachstehenden spezifischen Beispielen finden sich typische Versuchsprotokolle in denen die Fähigkeit der Testsubstanz untersucht wurde, als Malariamittel selbst gegen medikamentenresistente Stämme von P. berghei wirksam zu sein.

Die vorliegende, nachstehend ausführlicher beschriebene Kombination von Malariamitteln ermöglicht die gewünschte Malariabehandlung, und zwar sowohl zur Prophylaxe als auch zur Therapie und verhindert oder verzögert die Resistenzentstehung.

Artemisinin oder eines seiner vorstehend genannten Derivate wird bei Säugern im allgemeinen im Bereich von 0,125-10 mg/kg x 5 Tage täglich in einer Einzeldosis verabreicht. Das bereits weiter oben in dieser Spezifikation genannte weitere Malariamittel (d.h. Chloroquin, 10-O-Methylfloxacrin, Chinin, Pyrimethamin, Mefloquin, Amodiaquin, Sulfadoxin und Primaquin) kann getrennt verabreicht werden; in diesem Fall wird letzteres in einer Menge innerhalb des (jedoch meistens niedrigeren) Dosisbereichs und entsprechend der Behandlungsschemata (Häufigkeit, Darreichungsform und Zusammensetzungen) verabreicht, wie sie für deren Anwendungen in bisherigen Veröffentlichungen, beispielsweise in den oben zitierten Literaturquellen oder weiter in den genannten Literaturquellen spezifiziert werden.

Vorzugsweise und bequemer werden Artemisinin oder eines seiner Derivate und weitere Malariamittel der Erfindung in einer einzigen, kombinierten Zusammensetzung verabreicht. Dies kann eine Form sein, die sich zur parenteralen Verabreichung eignet, jedoch ist eine zur oralen Verabreichung geeignete Form vorzuziehen. Der Anteil jedes Medikamentes in der vorgeschlagenen kombinierten Dosierungsform entspricht dem Anteil der Tagesgesamtdosis jedes Medikamentes bei dessen alleiniger Verabreichung. Die kombinierten Medikamente können in Einzel-oder Teildosen verabreicht werden.

Bei der bevorzugten oralen Verabreichung wird die Menge an Artemisinin für einen durchschnittlichen, erwachsenen Patienten im allgemeinen im Bereich von 0,2-2 g in Kombination mit 200-400 mg Chloroquin oder mit 200-400 mg 10-O-Methylfloxacrin als erster Dosis liegen; die 2. Dosis kann 6 Stunden später im Bereich von 0,2-2 g Artemisinin in Kombination mit 100-200 mg Chloroquin oder mit 100-200 mg 10-O-Methylfloxacrin verabreicht werden. Die bei der zweiten Einnahme verabreichte Dosis kann weitere 3 Tage beibehalten werden, wobei täglich eine Einzeldosis verabreicht wird.

Kombinationen für Artemisinin oder eines seiner Derivate mit anderen Malariamitteln der zweiten Gruppe können in ähnlicher Weise verabreicht werden. Im algemeinen kann einem erwachsenen Patienten eine Kombination von Dihydroartemisinin (Bereich 0,2-1,5 g) mit Chloroquin (Bereich 200-400 mg) oder mit 10-O-Methylfloxacrin (Bereich 200-400 mg) verabreicht werden, wobei 6 Stunden später eine 2. Dosis von 0,2-1,5 g Dihydroartemisinin in Kombination mit 100-200 mg Chloroquin oder mit 100-200 mg 10-O-Methylfoxacrin verabreicht wird. Die als 2. Dosis verabreichte Menge kann im allgemeinen 3 weitere Tage

lang täglich als Einzeldosis verabfolgt werden.

Einem erwachsenen Patienten kann auch eine Kombination von Artether (Bereich 0,2-1,5 g) zusammen mit Chloroquin oder mit 10-O-Methylfloxacrin (Bereich 200-400 mg) verabreicht werden. Die 2. Dosis kann 6 Stunden nach der 1. Dosis verabreicht werden und kann 0,2-1,5 g Arteether plus 100-200 mg Chloroquin oder 10-O-Methylfloxacrin enthalten.

Die mit der 2. Dosis verabreichte Menge kann im allgemeinen weitere 3 Tage lang täglich als Einzeldosis gegeben werden.

Die kombinierten Substanzen werden alleine oder in einer weiteren Kombination mit pharmazeutisch anwendbaren Trägerstoffen sowohl oral wie parenteral verabreicht. Bei oraler Gabe zählen zu den geeigneten pharmazeutischen Trägerstoffen inerte Verdünnungs-oder Füllmittel, die zur Herstellung von Tabletten, Pulvern, Kapseln o.ä. verwendet werden. Diese pharmazeutischen Zusammensetzungen können, falls dies gewünscht wird, zusätzliche Bestandteile wie Aromastoffe, Bindemittel, Korrigentien o.ä. enthalten. Es werden beispielsweise Tabletten verwendet, die verschiedene Korrigentien wie Natriumzitrat zusammen mit verschiedenen löslichen Substanzen wie Stärke, Alginate und bestimmten komplexen Silikaten und Bindemitteln wie Polyvinylpyrrolidon, Suchrose, Gelatine und Gummi arabicum enthalten. Für das Herstellen von Tabletten eignen sich außerdem häufig gut Gleitmittel wie Magnesiumstearat, Natriumlaurylsulfat und Talkum. Feste Zusammensetzungen ähnlicher Art werden auch als Füllstoffe in gefüllten Weich-und Hartgelatinekapseln verwendt. Zu den bevorzugten Materialien zählen dehalb Laktose und Polyäthylenglykole mit hohem Molekulargewicht.

Die vorliegende Erfindung wird durch die nachstehenden Beispiele veranschaulicht. Es soll allerdings darauf hingeweisen werden, daß sich die Erfindung nicht auf die spezifische Einzelheiten der Beispiel beschränkt.

Beispiel 1

Synergistische therapeutische Wirkungen subkurativer Dosen von Artemisinin, Dihydroartemisinin und Artether in Kombination mit subkurativen Dosen von Chloroquin, 10-O-Methylfloxacrin, Mefloquin oder Pyrimethamin gegen die chloroquin-empfindliche Plasmodium berghei-Infektion bei Schweizer Mäusen.

Methodologie der biologischen Bewertung:

Hierzu diente die Beurteiling der schizontoziden Wirkung im Blut des von Raether und Fink (Ann. Trop. Med. and Parasit., 73 (1979), 503-526) beschriebenen "28-Tage-Tests".

Mäuse: Sämtliche Experimente wurden mit randomisiert gezüchteten männlichen und weiblichen Schweizer Mäusen durchgeführt, die aus der Aufzuchteinrichtung von Hoechst India Ltd. in Muland, Bombay stammten. Die Tiere waren frei von Eperythrozo0n cocoides. Die Tiere erhielten Trockenfutter und Wasser ad lib. und wurden bei einer Raumtemperatur von 22-25 °C gehalten.

Parasit: Die "London School of Hygiene and Tropical Medicines" lieferte den gegen Medikamente empfindlichen Stamm Plasmodium berghei K-173 und gegen Chloroquin mäßig resistentes P. berghei (NS). Die Stämme rufen bei mit $1x10^7$ Parasiten befallenen Erythrozyten pro Maus eine tödliche Infektion hervor, nachdem sie intraperitoneal inokuliert worden sind.

Verabreichung der Substanzen: Die Substanzen wurden nach den von Raether und Fink (a.a.O.) beschriebenen Methoden oral oder subkutan verabfolgt. Artemisinin, Dihydroartemisinin und Arteether wurden in doppelt raffiniertem Maisöl homogenisiert und als Suspensionen für die subkutane Inokulation bei Mäusen verwandt. Die Medikamente wurden 5 Tage lang verabreicht. Die 1. Gabe erfolgte innerhalb von 2 Stunden nach der Infektion (D+O), gefolgt von D+1, D+2, D+3 und D+4 (jeweils 1 Tagesintervall).

Beobachtung der behandelten Mäuse: Ab D+4 wurden in unterschiedlichen Intervallen bis D+28 Blutausstriche angefertigt. Die Blutausstriche stammten aus dem hinteren Schwanzende und wurden mit Giemsa-Lösung gefärbt. Die Mäuse waren am D+28 frei von P. berghei und galten als vollständig ausgeheilt. Für jede Dosierung wurden mindestens 12 Mäuse untersucht.

Die synergistische therapeutische Wirkung subkurativer Dosen von Artemisinin, Dihydroartemisinin und Arteether jeweils in Kombination mit subkurativen Dosen von Chloroquin, Mefloquin, Pyrimethamin oder 10-O-Methylfloxacrin auf die mit chloroquin-empfindlichen P. berghei infizierten Mäusen ist in Tabelle I für die

orale und in Tabelle II für die subkutane Verabreichung der Substanzen dargestellt. Wie diese Daten zeigen, heilen subkurative Dosen von Artemisinin, Dihydroartemisinin und Arteether jeweils in Kombination mit subkurativen Dosen von Chloroquin, 10-O-Methylfloxacrin oder Pyrimethamin mit chloroquin-sensiblen P. berghei infizierten Mäusen völlig, wenn die Substanzen entweder oral oder subkutan verabfolgt werden.

Tabelle 1

| Zusammensetzung | Orale Dosis (mg/kgx5) | Mäuse pro Gruppe | % geheilte infizierte Tiere |
|---|---|---|---|
| Chloroquin (kurative Dosis) | 12,5 | 25 | 100 |
| Chloroquin (subkurative Dosis) | 10 | 27 | 40 |
| 10-0-Methylfloxacrin (kurative Dosis) | 10 | 12 | 100 |
| 10-0-Methylfloxacrin (subkurative Dosis) | 5 | 20 | 55 |
| Mefloquin (kurative Dosis) | 7,5 | 20 | 100 |
| Mefloquin (subkurative Dosis) | 2,5 | 32 | 6 |
| Pyrimethamin (kurative Dosis) | 7,5 | 16 | 100 |
| Pyrimethamin (subkurative Dosis) | 1,25 | 16 | 18 |
| Artemisinin (kurative Dosis) | 200 | 12 | 100 |
| Artemisinin (subkurative Dosis) | 100 | 20 | 40 |
| Dihydroartemisinin (kurative Dosis) | 100 | 12 | 100 |
| Dihydroartemisinin (subkurative Dosis) | 50 | 12 | 50 |
| Arteether (kurative Dosis) | 100 | 12 | 91 |
| Arteether (subkurative Dosis) | 50 | 15 | 40 |
| Artemisinin + Chloroquin | 10+10 | 12 | 100 |
| Dihydroartemisinin + Chloroquin | 5+5 | 12 | 100 |
| Arteether + Chloroquin | 7,5+5 | 12 | 100 |
| Artemisinin + 10-0-Methyl-floxacrin | 20+5 | 12 | 100 |
| Arteether + 10-0-Methylfloxacrin | 10+5 | 12 | 100 |
| Dihydroartemisinin + 10-O-methylfloxacrin | 10+5 | 12 | 100 |
| Arteether + Mefloquin | 10+2,5 | 32 | 100 |
| Arteether + Pyrimethamin | 10+1,25 | 21 | 100 |

Tabelle 2

| Zusammensetzung | Subkutane Dosis (mg/kgx5) | Mäuse pro Gruppe | % geheilte infizierte Tiere |
|---|---|---|---|
| Chloroquin (kurative Dosis) | 10 | 25 | 100 |
| Chloroquin (subkurative Dosis) | 5 | 24 | 25 |
| 10-0-Methylfloxacrin (kurative Dosis) | 5 | 19 | 100 |
| 10-0-Methylfloxacrin (subkurative Dosis) | 2,5 | 13 | 84 |
| Pyrimethamin (kurative Dosis) | 6 | 15 | 100 |
| Pyrimethamin (subkurative Dosis) | 2,5 | 15 | 66 |
| Artemisinin (kurative Dosis) | 20 | 12 | 100 |
| Artemisinin (subkurative Dosis) | 1,0 | 12 | 25 |
| Dihydroartemisinin (kurative Dosis) | 4 | 12 | 100 |
| Arteether (kurative Dosis) | 5,0 | 16 | 100 |
| Arteether (subkurative Dosis) | 1,25 | 16 | 33 |
| Dihydroartemisinin (subkurative Dosis) | 2 | 12 | 100 |
| Arteether (kurative Dosis) | 5 | 16 | 100 |
| Arteether (subkurative Dosis) | 1,25 | 16 | 33 |
| Artemisinin + Chloroquin | 5+5 | 12 | 100 |
| Dihydroartemisinin + Chloroquin | 2,5+5 | 12 | 100 |
| Arteether + Chloroquin | 5+5 | 12 | 100 |
| Artemisinin + 10-O-Methylfloxacrin | 10+2,5 | 12 | 100 |
| Dihydroartemisinin + 10-O-Methylfloxacrin | 2,5+2,5 | 12 | 100 |
| Arteether + 10-O-Methylfloxacrin | 5+2,5 | 14 | 100 |
| Arteether + Pyrimethamin | 1,25+1,25 | 22 | 100 |

Beispiel 2

Synergistische Wirkung subkurativer Dosen von Artemisinin oder Artemisininderivaten in Kombination mit subkurativen Dosen von Chloroquin, 10-O-Methylfloxacrin oder Pyrimethamin gegen chloroquin-resistente Stämme von Plasmodium berghei (NS) bei infizierten Schweizer Mäusen.

Die Beurteilung der schizontoziden Wirkungen im Blut erfolgte nach dem in Beispiel 1 beschriebenen Verfahren unter Verwendung des chloroquin-resistenten Stammes von P. berghei.

Die synergistische therapeutische Wirkung subkurativer Dosen von Artemisinin, Dihydroartemisinin bzw. Arteether in Kombination mit subkurativen- Dosen von Chloroquin oder von 10-O-Methylfloxacrin oder Pyrimethamin gegen die Infektion von Schweizer Mäusen mit Plasmodium berghei (NS) ist in Tabelle III für die orale bzw. subkutane Verabreichung aufgeführt. Wie diese Daten zeigen, werden die mit chloroquin-resistentem P. berghei infizierten Mäuse mit subkurativen Dosen von Artemisinin, Dihydroartemisinin bzw. Arteether in Kombination mit subkurativen Dosen von Chloroquin oder 10-O-Methylfloxacrin oder Pyrimethamin völlig geheilt, wenn die Substanzen entweder oral oder subkutan verabreicht werden.

Tabelle 3

| Zusammensetzung | Route | Dosis (mg/kgx5) | Mäuse pro Gruppe | % geheilte infizierte Tiere |
|---|---|---|---|---|
| Chloroquin | oral | 40 | 24 | 50 |
| (subkurative Dosis) | s.c. | 20 | 24 | 50 |
| 10-O-Methylfloxacrin | oral | 10 | 17 | 52 |
| (subkurative Dosis) | s.c. | 2,5 | 15 | 73 |
| Pyrimethamin | oral | 5,0 | 16 | 100 |
| (kurative Dosis) | s.c. | 1,25 | 20 | 100 |
| Pyrimethamin | oral | 0,31 | 40 | 25 |
| (subkurative Dosis) | s.c. | 1,25 | 16 | 50 |
| Artemisinin | oral | 200 | 12 | 50 |
| (subkurative Dosis) | s.c. | 20 | 12 | 50 |
| Dihydroartemisinin | oral | 100 | 12 | 50 |
| (subkurative Dosis) | s.c. | 5 | 12 | 50 |
| Arteether | oral | 100 | 12 | 50 |
| (subkurative Dosis) | s.c. | 10 | 15 | 80 |
| Artemisinin + | oral | 25+15 | 12 | 100 |
| Chloroquin | s.c. | 10+15 | 15 | 100 |
| Dihydroartemisinin + | oral | 15+15 | 12 | 100 |
| Chloroquin | s.c. | 5+15 | 15 | 100 |
| Arteether + Chloroquin | oral | 10+15 | 12 | 100 |
| | s.c. | 5+15 | 15 | 100 |
| Artemisinin + | oral | 15+5 | 16 | 100 |
| 10-O-Methylfloxacrin | s.c. | 10+2,5 | 16 | 100 |
| Dihydroartemisinin + | oral | 15+5 | 20 | 100 |
| 10-O-Methylfloxacrin | s.c. | 2,5+2,5 | 16 | 100 |
| Arteether + | oral | 10+7,5 | 14 | 100 |
| 10-O-Methylfloxacrin | s.c. | 5+2,5 | 20 | 100 |
| Arteether + Pyrimethamin | oral | 20+0,31 | 22 | 100 |
| | s.c. | 5+0,31 | 22 | 100 |

## Ansprüche

1. Arzneimittelkombination mit synergistischer Wirkung gegen Malaria, dadurch gekennzeichnet, daß sie neben üblichen Hilfs-und Trägerstoffen eine oder mehrere Verbindungen der Formel I

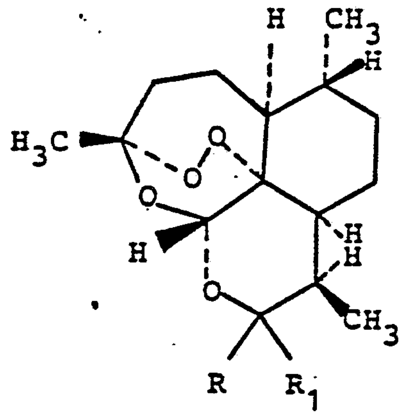

I

enthält, worin R und $R_1$ zusammen Sauerstoff, oder R jeweils Wasserstoff und $R_1$ OH, -O-$C_1$-$C_6$-Alkyl, -O-$C_1$-$C_6$-Alkenyl, -O-$C_1$-$C_6$-Alkanoyl, O-$C_1$-$C_6$-Alkanoylcarbonsäure, -O-Cyclohexylcarbonyl, -O-Benzoyl oder O-Naphtoyl bedeuten, sowie deren pharmakologisch verträglichen Salze (Gruppe 1)
und eine oder mehrere Verbindungen aus der Gruppe (2) Chloroquin, 10-O-Methylfloxacrin, Chinin, Mefloquin, Amodiaquin, Pyrimethamin, Sulfadoxin, Primaquin sowie deren pharmazeutisch wirksamen Salze enthält.

2. Arzneimittelkombination nach Anspruch 1, dadurch gekennzeichnet, daß sie eine oder mehrere Verbindungen aus der Gruppe Artemisinin, Dihydroartemisinin, Arteether, Artemether und Artesunat und eine oder mehrere Verbindungen aus der Gruppe (2) enthält.

3. Arzneimittelkombination nach Anspruch 1, dadurch gekennzeichnet, daß sie jeweils eine Verbindung aus der Gruppe (1) und eine oder mehrere Verbindungen aus der Gruppe (2) enthält.

4. Arzneimittelkombination nach Anspruch 1, dadurch gekennzeichnet, daß sie eine Verbindung aus Gruppe (1) und eine aus Gruppe (2) enthält.

5. Arzneimittelkombination nach Anspruch 1, dadurch gekennzeichnet, daß sie Artemisinin und Chloroquin enthält.

6. Arzneimittelkombination nach Anspruch 1, dadurch gekennzeichnet, daß sie Artemisinin und 10-O-Methylfloxacrin enthält.

7. Arzneimittelkombination nach Anspruch 1, dadurch gekennzeichnet, daß sie Artemisinin und Pyrimethamin enthält.

8. Arzneimittelkombination nach Anspruch 1, dadurch gekennzeichnet, daß sie Dihydroartemisinin und Chloroquin enthält.

9. Arzneimittelkombination nach Anspruch 1, dadurch gekennzeichnet, daß sie Dihydroartemisinin und 10-O-Methylfloxacrin enthält.

10. Arzneimittelkombination nach Anspruch 1, dadurch gekennzeichnet, daß sie Dihydroartemisin und Pyrimethamin enthält.

11. Arzneimittelkombination nach Anspruch 1, dadurch gekennzeichnet, daß sie Arteether und Chloroquin enthält.

12. Arzneimittelkombination nach Anspruch 1, dadurch gekennzeichnet, daß sie Arteether und 10-O-Methylfloxacrin enthält.

13. Arzneimittelkombination nach Anspruch 1, dadurch gekennzeichnet, daß sie Erteether und Pyrimethamin enthält.

14. Verwendung einer Kombination von Verbindungen der Gruppe (1) und (2) nach Anspruch 1 zur Herstellung von Arzneimitteln mit synergistischer Wirkung gegen Malaria.

15. Verwendung einer Kombination von Verbindungen nach Anspruch 2, zur Herstellung von Arzneimitteln mit synergistischer Wirkung gegen Malaria.

16. Verwendung einer Kombination von Verbindungen nach Anspruch 14, dadurch gekennzeichnet, daß sie jeweils eine Verbindung aus der Gruppe (1) und eine oder mehrere aus der Gruppe (2) enthält.

17. Verwendung einer Kombination von Verbindungen nach Anspruch 14, dadurch gekennzeichnet, daß sie jeweils eine Verbindung aus der Gruppe (1) und eine aus der Gruppe (2) enthält.

18. Verwendung einer Kombination von Verbindungen nach Anspruch 14, dadurch gekennzeichnet, daß sie Artemisinin und Chloroquin enthält.

19. Verwendung einer Kombination von Verbindungen nach Anspruch 14, dadurch gekennzeichnet, daß sie Artemisinin und 10-O-Methylfloxacrin enthält.

20. Verwendung einer Kombination von Verbindungen nach Anspruch 14, dadurch gekennzeichnet, daß sie Artemisin und Pyrimethamin enthält.

21. Verwendung einer Kombination von Verbindungen nach Anspruch 14, dadurch gekennzeichnet, daß sie Dihydroartemisinin und Chloroquin enthält.

22. Verwendung einer Kombination von Verbindungen nach Anspruch 14, dadurch gekennzeichnet, daß sie Dihydroartemisinin und 10-O-Methylfloxacrin enthält.

23. Verwendung einer Kombination von Verbindungen nach Anspruch 14, dadurch gekennzeichnet, daß sie Dihydroertemisin und Pyrimethamin enthält.

24. Verwendung einer Kombination von Verbindungen nach Anspruch 14, dadurch gekennzeichnet, daß sie Arteether und Chloroquin enthält.

25. Verwendung einer Kombination von Verbindungen nach Anspruch 14, dadurch gekennzeichnet, daß sie Arteether und 10-0-Floxacrin enthält.

26. Verwendung einer Kombination von Verbindungen nach Anspruch 14, dadurch gekennzeichnet, daß sie Arteether und Pyrimethamin enthält.

Patentansprüche für folgende Vertragsstaaten: ES,GR

1. Verfahren zur Herstellung einer Arzneimittelkombination mit synergistischer Wirkung gegen Malaria, dadurch gekennzeichnet, daß eine oder mehrere Verbidnungen der Formel I

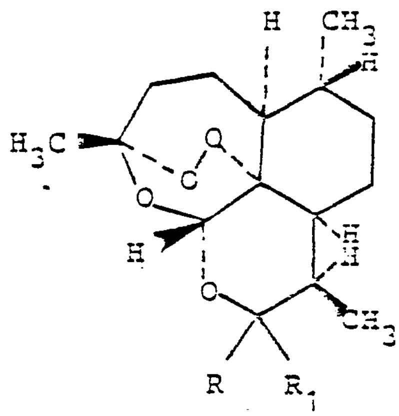

I

worin R und $R_1$ zusammen Sauerstoff, oder R jeweils Wasserstoff und $R_1$ OH, -O-$C_1$-$C_6$-Alkyl, -O-$C_1$-$C_6$-Alkenyl, -O-$C_1$-$C_6$-Alkanoyl, O-$C_1$-$C_6$-Alkanoylcarbonsäure, -O-Cyclohexylcarbonyl, -O-Benzoyl oder O-Naphthoyl bedeuten sowie deren pharmakologisch verträglichen Salze (Gruppe 1) und eine oder mehrere Verbindungen aus der Gruppe (2) Chloroquin, 10-O-Methylfloxacrin, Chinin, Mefloquin, Amodiaquin, Pyrimethamin, Sulfadoxin, Primaquin sowie deren pharmazeutisch wirksamen Salze mit üblichen Hilfs-und Trägerstoffen in eine geeignete Darreichungsform gebracht werden.

2. Verfahren zur Herstellung einer Arzneimittelkombination nach Anspruch 1, dadurch gekennzeichnet, daß eine oder mehrere Verbindungen aus der Gruppe Artemisinin, Dihydroartemisinin, Arteether, Artemether und Artesunat und eine odere mehrere Verbindungen aus der Gruppe (2) verwendet werden.

3. Verfahren zur Herstellung einer Arzneimittelkombination nach Anspruch 1, dadurch gekennzeichnet, daß jeweils eine Verbindung aus der Gruppe (1) und eine oder mehrere Verbindungen aus der Gruppe (2) verwendet werden.

4. Verfahren zur Herstellung einer Arzneimittelkombination nach Anspruch 1, dadurch gekennzeichnet, daß eine Verbindung aus Gruppe (1) und eine aus Gruppe (2) verwendet wird.

5. Verfahren zur Herstellung einer Arzneimittelkombination nach Anspruch 1, dadurch gekennzeichnet, daß Artemisinin und Chloroquin verwendet werden.

6. Verfahren zur Herstellung einer Arzenimittelkombination nach Anspruch 1, dadurch gekennzeichnet, daß Artemısinin und 10-O-Methylfloxacrin verwendet werden.

7. Verfahren zur Herstellung einer Arzneimittelkombination nach Anspruch 1, dadurch gekennzeichnet, daß Artemisinin und Pyrimethamin verwendet werden.

8. Verfahren zur Herstellung einer Arzneimittelkombination nach Anspruch 1, dadurch gekennzeichnet, daß Dihydroartemisinin und Chloroquin verwendet werden.

9. Verfahren zur Herstellung einer Arzneimittelkombination nach Anspruch 1, dadurch gekennzeichnet, daß Dihydroartemisinin und 10-O-Methylfloxacrin verwendet werden.

10. Verfahren zur Herstellung einer Arzneimittelkombination nach Anspruch 1, dadurch gekennzeichnet, daß Dihydroartemisin und Pyrimethamin verwendet werden.

11. Verfahren zur Herstellung einer Arzneimittelkombination nach Anspruch 1, dadurch gekennzeichnet, daß Arteether und Chloroquin verwendet werden.

12. Verfahren zur Herstellung einer Arzneimittelkombination nach Anspruch 1, dadurch gekennzeichnet, daß Arteether und 10-O-Methylfloxacrin verwendet werden.

13. Verfahren zur Herstellung einer Arzneimittelkombination nach Anspruch 1, dadurch gekennzeichnet, daß Arteether und Pyrimethamin verwendet werden.